(19) Europäisches Patentamt
European Patent Office
Office européen des brevets

(11) Veröffentlichungsnummer: **0 212 409**
**B1**

(12) # EUROPÄISCHE PATENTSCHRIFT

(45) Veröffentlichungstag der Patentschrifft: **21.03.90**

(51) Int. Cl.[5]: **C 07 D 317/36,** C 08 G 59/00

(21) Anmeldenummer: **86110736.5**

(22) Anmeldetag: **04.08.86**

(54) **Verfahren zur Herstellung von 2-oxo-1,3-dioxolanen.**

(30) Priorität: **16.08.85 DE 3529263**

(43) Veröffentlichungstag der Anmeldung:
**04.03.87 Patentblatt 87/10**

(45) Bekanntmachung des Hinweises auf die Patenterteilung:
**21.03.90 Patentblatt 90/12**

(84) Bennante Vertragsstaaten:
**AT BE CH DE FR GB IT LI NL SE**

(56) Entgegenhaltungen:
**EP-A-0 119 840**
**EP-A-0 149 156**
**GB-A-1 485 925**
**US-A-2 994 705**

**CHEMICAL ABSTRACTS, Band 62, 1965, Zusammenfassung Nr. 6395b,c, Columbus, Ohio, US; & SU-A-165 694 (S.Z. LEVIN et al.) 26-10-1964, & SU-A-165 435 (S.Z LEVIN et al.) 12-10-1964**
**PATENT ABSTRACTS OF JAPAN, Band 6, Nr. 102 (C-107) 980 , 11. Juni 1982; & JP-A-57 31 682 (NIPPON SHIKUBAI KAGAKU KOGYO K.K.) 20-02-1982**
**CHEMICAL ABSTRACTS, Band 77, 1972, Seite 392, Zusammenfassung Nr. 139417z, Columbus, Ohio, US; & JP-A-72 36 738 (MITSUI PETROCHEMICAL INDUSTRIES LTD) 14-09-1972**
**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Brindöpke, Gerhard, Dr.**
**Loreleistrasse 18**
**D-6230 Frankfurt am Main 80 (DE)**
Erfinder: **Marten, Manfred**
**Schneeheide 36**
**D-2104 Hamburg 92 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 212 409 B1

LIBERGRAF, STOCKHOLM 1990

**Beschreibung**

Es ist bekannt, daß man durch Umsetzung von Alkylenoxiden mit Kohlendioxid in Gegenwart von Katalysatoren 2-Oxo-1,3-dioxolane (auch Alkylencarbonate genannt) erhalten kann. In der DE-OS-2 611 087 wird ein Verfahren zur Herstellung von Alkylencarbonaten der allgemeinen Formel

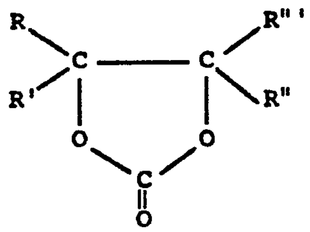

(1)

beschrieben, wobei ein Alkylenoxid mit $CO_2$ in Gegenwart eines Katalysators, der aus einer Kombination einer protischen Substanz der Formel ROH und einer stickstoffhaltigen Base besteht, bei Temperaturen zwischen 0 und 200°C und einem Druck von 1 bis 98 bar umgesetzt wird. Protische Substanzen sind Wasser, Alkohole und Phenol. Als stickstoffhaltige Basen werden Trimethylamin, Triethylamin, Pyridin oder Dimethylanilin angeführt. Bezüglich der Substituenten R bis R''' wird nur allgemein angegeben, daß diese Wasserstoff oder ein Alkyl-, Aryl-, Cycloalkyl- oder Aralkylrest sein können. Nähere Angaben werden nicht gemacht. In den Beispielen werden nur Ethylenoxid und Propylenoxid als Alkylenoxide angeführt und es wird stets unter Druck gearbeitet (minimal 10 bar).

G. Rokicki und Mitarbeiter beschreiben in "Monatshefte für Chemie" 115 (1984), 205 - 214, die Herstellung von cyclischen Carbonaten aus $CO_2$ und Oxiranen in Gegenwart von Alkalimetallsalz-Phasentransfer-Katalysatoren. Als Phasentransfermittel werden Kronenether, 1,4-Diazabicyclo[2,2,2]-octan (DABCO), N,N,N,N-Tetramethylethylendiamin (TMEDA) und Triethylbenzylammoniumchlorid (TEBA) aber auch - mit verringerter Ausbeute - Polyethylenglykol eingesetzt. Hohe Ausbeuten, d.h. fast theoretische Werte, werden nur durch Anfangsdrucke von 40 bar erreicht. Beim Arbeiten bei 6 bar wird eine um ca. 25 % niedrigere Ausbeute, bei 1 bar sogar nur 8 % Ausbeute erhalten. Als Epoxidkomponenten werden Ethylen- und Propylenoxid, Epihalogenhydrine, Glycid, n-Butyl-, Allyl- und Phenylglycidether, Styroloxid und 3,3-disubstituiertes Cyclohexenoxid angeführt.

Eine weitere Arbeit von G. Rokicki (Makromol. Chem. 186, 331 - 337 (1985)) beschreibt die Herstellung von cyclischen Dicarbonaten durch Einsatz von 2,2-Bis[4-(2,3-epoxypropoxy) phenyl]propan bzw. einem Epoxidharz (®Epikote 828) unter den vorstehend angegebenen Bedingungen.

Es ist auch bekannt (PCT WO 84/03,701), Alkylencarbonate durch Behandlung von Alkylenoxiden mit $CO_2$ in Gegenwart eines Alkohols wie Methanol und einer (un-)substituierten Phosphinverbindung als Katalysator herzustellen. Auch in diesem Falle wird bei erhöhtem Druck (21 bar) gearbeitet. Darüberhinaus ergibt sich aus der Veröffentlichung, daß die Gegenwart sowohl des Alkohols als auch des Phosphins zwinged sein muß, um eine gute Ausbeute zu erhalten.

Zum Stand der Technik gehören auch bereits quartäre Ammoniumverbindungen sowie Dimethylbenzylamin und Hydrazin (SU-PS-165 435) sowie quartäre Phosphoniumverbindungen (US-2 994 705). Zu beiden Fällen ist insbesondere der Anteil an überwiegend oligomeren/polymeren Nebenprodukten recht hoch, d.h. die Selektivität dieser Katalysatoren ist nicht ausreichend. Aus den Beispielen der US-PS entnimmt der Fachmann außerdem, daß Ausbeuten von 90 % oder mehr hiernach offensichtlich nur bei Drucken von mindestens 70 bar möglich sind.

Aus der JP-Patentameldung 5 731 682 ist schließlich noch die Verwendung von Alkalihalogeniden für derartige Carbonatisierungsreaktionen bekannt, wobei jedoch ein Alkylencarbonat - nach Lage der Dinge das gleiche wie das herzustellende - als Verdünnungsmittel dient. Die dabei erhältlichen Ausbeuten sind recht gering. Außerdem ist dieses Verfahren bei höhermolekularen Epoxiden praktisch kaum einsetzbar.

Aus dem Stand der Technik ergibt sich also, daß zur Erzielung einer hohen Ausbeute bei hohen Drucken gearbeitet werden muß, die Gegenhart einer protischen Substanz zwingend ist, um zufriedenstellende Ausbeuten zu erhalten und/oder die Selektivität der Verfahren zu gering ist.

Die genannten Nachteile können gemäß der vorliegenden Erfindung umgangen werden.

Gegenstand der Erfindung ist daher ein Verfahren zur Herstellung von 2-Oxo-1,3-dioxolanen durch Umsetzung von Epoxiden mit Kohlendioxid in Gegenwart eines Katalysators und gegebenenfalls eines inerten Lösungsmittels, dadurch gekennzeichnet, daß die Umsetzung bei Temperaturen von 40 bis 180°C und bei Normaldruck oder geringfügig erhöhtem Druck in Gegenwart mindestens eines Katalysators aus der Gruppe Triphenylphosphan, Tritolylphosphan, Diazybicyclo[2.2.2]octan, 4-Dimethylaminopyridin, 4-(1-Pyrrolidinyl)pyridin, Amidine, Imidazol und dessen Alkyl-, Aryl- oder Aralkyl-Substitutionsprodukten erfolgt.

Der Vorteil des erfindungsgemäßen Verfahrens liegt u.a. in der Anwendung von Normal- oder schwach erhöhtem Druck, wobei kein großer apparativer Aufwand erforderlich ist. Weiterhin ist die hohe Selektivität der Umsetzung hervorzuheben, d.h. es treten praktisch keine Epoxid-Nebenreaktionen wie Homopolymerisation ein, die bei diesem Reaktionsmechanismus im Stand der Technik beschrieben worden sind. Ferner ist es durch das Verfahren möglich, lagerstabile Epoxid-/Carbonatgemische herzustellen, die eine Multifunktionalität aufweisen und für viele Anwendungsgebiete verfügbar sind.

Der bei dem erfindungsgemäßen Verfahren anzuwendende Druck beträgt im allgemeinen 1 bis 10, vorzugsweise 1 bis 5 und insbesondere 1 bis 3 bar. In den meisten Fällen wird Normaldruck angewandt, gegebenenfalls kann aber auch unter erhöhtem Druck gearbeitet werden. Der bevorzugte Temperaturbereich des Verfahrens liegt bei 50 bis 160, insbesondere 60 bis 145°C.

Als Epoxidkomponenten, die mit $CO_2$ umgesetzt werden können und im allgemeinen mindestens eine endständige Epoxidgruppe besitzen, eigen sich beispielsweise folgende Verbindungen: aliphatische Epoxide mit mindestens 6 C-Atomen, wie Hexen-, Octen, Dodecen-1-oxid, Glycidol und Epihalogenhydrine der Formel

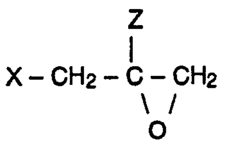

(2),

in der

Z ein Wasserstoffatom, eine Methyl- oder Ethylgruppe und
X ein Halogenatom oder eine OH-Gruppe darstellt.

Beispiele für derartige Epihalogenhydrine sind Epichlorhydrin, Epibromhydrin, 1,2-Epoxi-2-methyl-3-chlorpropan und 1,2-Epoxi-2-ethyl-3-chlorpropan.

Weitere Epoxykomponenten, die erfindungsgemäß eingesetzt werden können, schließen z. B. Epoxykomponenten, die im Durchschnitt mindestens eine endständige 1,2-Epoxygruppe aufweisen ein. Vorzugsweise sind dies Epoxyverbindungen, die im Durchschnitt mindestens eine substituierte oder nicht substituierte Glycidylethergruppe oder eine substituierte oder nicht substituierte Glycidylestergruppe enthalten, ferner epoxydierte, mehrfach ungesättigte Verbindungen und amid- oder urethangruppenhaltige Epoxide.

Epoxyverbindungen, die im Durchschnitt mindestens eine substituierte oder nicht substituierte Glycidylethergruppe enthalten, welche die Formel

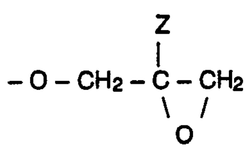

(3),

aufweist, in der Z Wasserstoff, eine Methyl- oder eine Ethylgruppe darstellt, sind z. B. Glycidyl- oder Polyglycidylether von Phenol oder mehrwertigen Phenolen, die ein oder mehrere aromatische Kerne aufweisen sowie von Novolaken, Polyglycidylether von alkoholischen Polyhydroxylverbindungen, erhalten durch Additionsreaktion von mehrwertigen Phenolen, enthaltend einen oder mehrere aromatische Kerne mit Alkylenoxiden, die 2 bis 4 C-Atome aufweisen, und Polyglycidylether von alkoholischen Polyhydroxylverbindungen, die einen oder mehreren alicyclische Ringe aufweisen. Als Phenole werden beispielsweise eingesetzt Phenol, die verschiedenen Kresole, Resorcin, Hydrochinon, Pyrogallol, Phloroglucin, 1,5-, 2,7-, 2,6-Dihydroxynaphthaline und ähnliche, 2,2-Bis-(p-hydroxyphenyl)-propan und -methan (bekannt als Bisphenol A bzw. F), 2,4'-Dihydroxydiphenylmethan u.ä.. Mehrwertige Alkohole, die zu Glycidylethern umgesetzt werden können, sind beispielsweise Ethylenglykol, Propylenglykol, Butylglykol, Neopentylglykol, Hexylenglykol, Polyethylenglykol, Polypropylenglykol u.ä..

Hierzu werden auch plastifizierte Epoxidharze mit endständigen Epoxygruppen gerechnet, die durch Teilumsetzung der Epoxygruppen von mindestens zwei Epoxygruppen enthaltenden Epoxidharzen mit OH- und COOH-haltigen Substanzen wie mehrwertigen Alkoholen, z. B. den obengenannten Diolen, Polycarbonsäuren oder Carboxyl- oder OH-gruppenhaltigen Polyestern hergestellt werden.

Weitere Epoxyverbindungen sind Glycidylester von gesättigten oder ethylenisch ungesättigten Carbonsäuren mit mindestens einer substituierten oder nicht substituierten Glycidylestergruppe der folgenden Formel

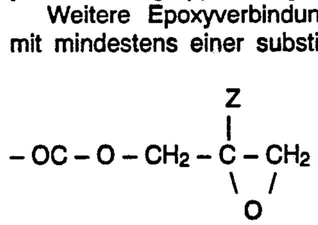

(4),

in der Z ein Wasserstoffatom, eine Methyl- oder eine Ethylgruppe darstellt. Die Säuren sind aliphatische oder aromatische, gesättigte oder ungesättigte Mono- oder Polycarbonsäuren, z. B. Acrylsäure, Methacrylsäure, Adipinsäure, die verschiedenen Phthalsäuren, Tetra- und Hexahydrophtalsäure u.ä.. Ein sehr gebräuchlicher Glycidylester ist im Handel erhältlich und stellt den Glycidylester einer Mischung von gesättigten Monocarbonsäuren mit einer Kettenlänge von 9 bis 11 Kohlenstoffatomen dar, bestehend hauptsächlich (ungefähr 94 %) aus tertiären Säuren (Versaticsäureglycidylester). Hier eingeschlossen sind auch Epoxidharze, die durch Copolymerisation von Glycidylmethacrylsäureester mit anderen copolymerisierbaren Monomeren wie Styrol und (Meth-)Acrylsäureestern erhalten worden sind. Des weiteren sind amid- oder urethangruppenhaltige Epoxide für die Umsetzung geeignet, z. B. Triglycidylisocyanurat oder glycidolverkapptes Hexamethylendiisocyanat. Mischungen der genannten Epoxidverbindungen können ebenfalls eingesetzt werden.

Erfindungsgemäß einsetzbare Katalysatoren sind beispielsweise die Phosphane aus der Gruppe Triphenylphosphan und Tritolylphosphan. Weitere Katalysatoren sind Diazabicyclo[2.2.2]octan (DABCO), 4-Dimethylaminopyridin (DMAP), 4-(1-Pyrrolidinyl)-pyridin, Amidine, wie 1,5-Diaza-bicyclo[4.3.0]non-5-en (DBN), 1,8-Diazabicyclo[5.4.0]undec-7-en (DBU) u.a., sowie Imidazol und dessen Alkyl-Substitutionsprodukte in 1-, 2-, 4- und 5-Stellung, mit bis zu 4 C-Atomen im Alkylrest, wie 1-Methylimidazol, 2-Methylimidazol, 2-Ethyl-4-methylimidazol u.a., aryl- und aralkylsubstituierte Imidazole, wie 2-Phenylimidazol, 2-Phenyl-4-methylimidazol u.a..

Die Katalysatoren werden im allgemeinen allein oder im Gemisch in Mengen von 0,02 bis 10, vorzugsweise 0,05 bis 6 und insbesondere 0,05 bis 3 Gewichtsprozent, bezogen auf das Gewicht der Epoxidkomponente, eingesetzt.

Die Reaktionszeit kann in weiten Grenzen schwanken. Im allgemeinen wird die Umsetzung so geführt, daß die Epoxidgruppen praktisch vollkommen umgesetzt sind. Die Reaktion wird beispielsweise durch Titration der Epoxidgruppen verfolgt und an dem Punkt abgebrochen, der im Rahmen der Analysengenauigkeit als "epoxidgruppenarm bzw. -frei" angesehen wird. Man erhält auf diese Weise Alkylcarbonate aus beliebigen Epoxidverbindungen, die in bekannter Weise weiterverarbeitet werden können.

Darüberhinaus kann man die Reaktion bei Vorliegen von Polyepoxiden an jedem gewünschten Punkt abbrechen, so daß Verbindungen erhalten werden, die neben Carbonatgruppen noch intakte Epoxidgruppen aufweisen. Letzteres hat den Vorteil, daß je nach Art der gewünschten Weiterverarbeitung und des Einsatzes der Produkte eine selektive Umsetzung der Epoxid- neben der Carbonatgruppe - und umgekehrt - erfolgen kann. Im allgemeinen wird man bei den Polyepoxiden (Anzahl der Epoxidgruppen $n \geq 2$) die Umsetzung in diesem Fall so durchführen, daß der Anteil der umgesetzten Epoxidgruppen 0,1 - 0,9 n beträgt.

Man kann die Wirkung der Katalysatoren verbessern, indem man als Co-Katalysatoren Halogenide oder Carbonate von Alkali- oder Erdalkalimetallen in Mengen von 0,1 bis 10, vorzugsweise 0,2 bis 5 und insbesondere 0,4 bis 2,5 Gew.-%, bezogen auf die Epoxidkomponente, den Ansätzen zugibt. Hierbei können die Chloride, Bromide, Jodide oder Carbonate der Metalle Lithium, Natrium, Kalium, Magnesium und Calcium verwendet werden, wobei KJ und NaJ bevorzugt sind. Die Co-Katalysatoren werden im allgemeinen aber nur dann eingesetzt, wenn eine Reaktivitätssteigerung erwünscht ist und sie darüberhinaus die Verwendbarkeit nicht beeinträchtigen und durch einfache Operationen abgetrennt werden können.

Die Umsetzung der Epoxidverbindungen mit $CO_2$ kann in Anwesenheit oder Abwesenheit von Lösungsmitteln erfolgen. Im allgemeinen werden keine Lösungsmittel eingesetzt, wenn die Epoxidverbindungen oberhalb 50°C im flüssigen Zustand vorliegen. Stellen sie jedoch bei der Reaktionstemperatur zähflüssige Schmelzen dar und erschweren dadurch eine homogene Verteilung des Kohlendioxids beim Rühren oder ist eine Weiterverarbeitung des Reaktionsproduktes in Lösung vorgesehen, werden im allgemeinen Lösungsmittel verwendet.

Als Lösungsmittel können aromatische Kohlenwasserstoffe wie Toluol, Xylol und bei der Erdölcrackung anfallende Kohlenwasserstoffgemische, ferner Ether wie Dioxan, Tetrahydrofuran, Glykol- und Diglykoldimethylether und andere gegen Epoxigruppen inerte Lösungsmittel eingesetzt werden.

In den nachfolgenden Beispielen bedeutet T stets Gewichtsteile und % stets Gewichts-%.

**Beispiele**

**Allgemeine Vorschrift zur Herstellung von Biscarbonaten aus Diglycidylethern des Bisphenol A, Beispiele 1 bis 22**

In einer mit Rührer, Thermometer und einem Gaseinleitungsrohr bestückten Apparatur (gegebenenfalls Druckapparatur) wurde ein technischer Diglycidylether des Bisphenol A mit dem in der Tabelle angeführten Epoxidgehalt vorgelegt, gegebenenfalls gelöst in dem angegebenen Lösungsmittel, und nach Zugabe des Katalysators und gegebenenfalls des Co-Katalysators unter Rühren und unter Einleiten von Kohlendioxid auf die angegebene Reaktionstemperatur erhitzt. Beim Arbeiten unter Druck wurde die Reaktionsapparatur vorher mit Kohlendioxid ausgespült. Als Glycidylether wurden "®Beckopox EP 140" (Epoxidgehalt 8,6 %) und "Beckopox EP 301" (Epoxidgehalt 3,3 %) der Fa. Hoechst AG eingesetzt.

Unter fortlaufendem Einleiten von Kohlendioxid wurde bei den genannten Reaktionstemperaturen und Drücken bis zum gewünschten Restepoxidgehalt, der durch Titration ermittelt wird, nachgerührt. Anschließend wurde bei Verwendung unlöslicher Katalysatoren in der Hitze filtriert und anwesendes Lösungsmittel unter vermindertem Druck gegebenenfalls abdestilliert.

In den Beispielen 1 - 4 wird die Herstellung von carbonatgruppenhaltigen Epoxidharzen durch teilweise Umsetzung von Diglycidylethern des Bisphenol A beschrieben. Die Beispiele 21 und 22 stellen Vergleichsversuche gemäß dem Stand der Technik dar.

Die nach den Beispielen 5 bis 20 hergestellten Biscarbonate können aus Methoxypropanol umkristallisiert werden; z. B. hatte das Produkt von Beilspiel 10 nach Umkristallisation folgende Eigenschaften:

| | | |
|---|---|---|
| IR-Absorption: | 1790 cm | |
| Elementaranalyse: | $C_{gef.}$ 64,4 | $C_{ber}$ 64,5 |
| | $H_{gef.}$ 5,8 | $H_{ber.}$ 5,6 |
| Carbonatgehalt an gebundenem $CO_2$: | 18,6 %. | |

In der Tabelle 1 bedeutet:

| | |
|---|---|
| DABCO: | 1,4-Diazabicyclo-[2.2.2]-octan |
| DBN: | Diazabicyclo-[4.3.0]-nonen-(5) |
| DGDME: | Diglykoldimethylether |
| DMAP: | 4-Dimethylaminopyridin |
| TPP: | Triphenylphosphan |
| TTP: | Tritolylphosphan |

**Tabelle 1**

| Beispiel | Diglycidylether Epoxidgeh. (%) | Menge (Teile) | Katalysator | Menge (Teile) | % | Co-Katalysator | Menge (Teile) | % |
|---|---|---|---|---|---|---|---|---|
| 1 | 8.6 | 30000 | TPP | 380 | 1,3 | - | - | - |
| 2 | 8.6 | 1000 | TPP | 2 | 1,2 | KJ | 9 | 0,9 |
| 3 | 8.6 | 1716 | DABCO | 4 | 0,23 | - | - | - |
| 4 | 8.6 | 1488 | TTP | 9,8 | 0,66 | - | - | - |
| 5 | 8.6 | 186 | TPP | 2,5 | 1,34 | NaJ | 1,1 | 0,6 |
| 6 | 8.6 | 186 | TPP | 2,6 | 1,4 | LiJ | 2,3 | 1,23 |
| 7 | 8.6 | 186 | TPP | 2,6 | 1,4 | $CaCl_2$ | 1,5 | 0,8 |
| 8 | 8.6 | 186 | DABCO | 1,0 | 0,5 | KJ | 2,0 | 1,1 |
| 9 | 8.6 | 186 | TPP | 3 | 1,6 | NaBr | 2,0 | 1,1 |
| 10 | 8.6 | 186 | TPP | 2,6 | 1,4 | KJ | 1,1 | 0,6 |
| 11 | 8.6 | 30000 | TPP | 400 | 1,3 | KCl | 210 | 0,7 |
| 12 | 8.6 | 1860 | TTP | 27 | 1,45 | NaJ | 10 | 0,55 |
| 13 | 8.6 | 1000 | TPP | 7 | 0,7 | NaJ | 4 | 0,4 |
| 14 | 8.6 | 1412 | DBN | 3,76 | 0,27 | - | - | |
| 15 | 8.6 | 1180 | DMAP | 3,1 | 0,26 | - | - | |
| 16 | 8.6 | 1370 | 2-Ethyl-4-methylimidazol | 3,2 | 0,23 | - | - | |
| 17 | 8.6 | 1277 | " | 3,0 | 0,23 | - | - | |
| 18 | 8.6 | 1338 | 1-Methylimidazol | 2,9 | 0,2 | - | - | |
| 19 | 8.6 | 1185 | 2-methylimidazol | 2,1 | 0,2 | - | - | |
| 20 | 8.6 | 1225 | Imidazol | 1,8 | 0,15 | - | - | |
| 21 (V1) | 8.6 | 1315 | Triethylamin | 9 | 0,7 | - | - | |
| 22 (V2) | 8.6 | 1286 | Triethylamin | 39 | 3,0 | - | - | |

**Tabelle 1**, Forts.

| Beispiel | Lösungsmittel | Menge (Teile) | Temp. (°C) | Druck (bar) | Reaktionszeit (h) | Ausbeute % | Ausbeute (Teile) | Schmelzpunkt ($F_p$) | Epoxidgehalt (%) | Gehalt an geb. $CO_2$ (%) |
|---|---|---|---|---|---|---|---|---|---|---|
| 1 | - | - | 130 | 2 | 16 | | 33700 | - | 3,8 | 10,2 |
| 2 | Xylol | 200 | 120 | 1 | 7 | | 1080 | - | 4,2 | 9,4 |
| 3 | - | - | 120 | 1 | 16 | | 1908 | - | 2,7 | 12,5 |
| 4 | n-Buthanol | 372 | 118 | 1 | 12 | - | 1585 | - | 3,5 | 11,1 |
| 5 | - | - | 120 | 1 | 17 | 97,9 | 224 | 52 - 53 | 0,2 | 18,5 |
| 6 | - | - | 120 | 1 | 19 | 96,2 | 220 | 51 - 52 | 0,2 | 18,5 |
| 7 | - | - | 140 | 1 | 29 | 96,5 | 219 | 48 - 50 | 0,5 | 18,0 |
| 8 | - | - | 120 | 1 | 14 | 97,8 | 223 | 51 - 52 | 0,3 | 18,5 |
| 9 | - | - | 130 | 1 | 24 | 96,9 | 221 | 50 - 52 | 0,3 | 18,1 |
| 10 | - | - | 120 | 1 | 18 | 97,9 | 224 | 50 - 52 | 0,2 | 18,6 |
| 11 | - | - | 130 | 2 | 26 | 95,5 | 35790 | 49 - 51 | 0,4 | 18,2 |
| 12 | - | - | 130 | 1 | 27 | 95,8 | 2180 | 51 - 53 | 0,4 | 18,1 |
| 13 | DGDME | 300 | 120 | 1 | 12 | 97,8 | 1205 | 50 - 51 | 0,2 | 18,4 |
| 14 | - | - | 120 | 1 | 16 | 96,8 | 1685 | 51 - 52 | 0,1 | 18,1 |
| 15 | DGDME | 10 | 100 | 1 | 15 | 92,8 | 1350 | 125 - 138 | 0,1 | 18,7 |
| 16 | DGDME | 15 | 100 | 1 | 15 | 92,0 | 1550 | 112 - 131 | 0,2 | 18,4 |
| 17 | - | - | 100 | 1 | 16 | 91,2 | 1420 | 49 - 52 | 0,5 | 17,8 |
| 18 | DGDME | 10 | 100 | 1 | 18 | 90,2 | 1480 | 51 - 53 | 0,3 | 18,2 |
| 19 | - | 10 | 100 | 1 | 17 | 91,8 | 1320 | 49 - 51 | 0,7 | 17,3 |
| 20 | - | - | 120 | 1 | 17 | 95,5 | 1438 | 52 - 55 | 0,2 | 18,3 |
| 21 (V1) | n-Butanol | 329 | 120 | 1 | 16 | - | - | - | 0,5 | - |
| 22 (v2) | n-Butanol | 322 | 120 | 1 | Gelation nach 1 h | | | | | |

**Beispiele 23 bis 31**

23) 142 T Glycidylmethacrylat, 1,3 T Triphenylphosphan und 0,6 T Kaliumjodid wurden in Gegenwart von 0,3 T Hydrochinonmonomethylether auf 80°C erhitzt. Unter Durchleitung von Kohlendioxid wurde 17 Stunden nachgerührt. Nach Filtrieren und Abkühlen wurden 181 T (98,1 %) einer klaren gelben viskosen Flüssigkeit erhalten. Epoxidgehalt: 0,3 %,

| | | |
|---|---|---|
| Elementaranalyse: | C gef.: 51,3 % | C ber.: 51,6 % |
| | H gef.: 5,6 % | H ber.: 5,4 % |
| Siedepunkt: | 135 - 137°C / 0,1 - 0,2 Torr | |

42) 142 T Glycidylmethanacrylat, 0,7 T Diazabicyclooctan und 0,5 T Lithiumjodid wurden in Gegenwart von 0,3 T Hydrochinonmonomethylether auf 80°C erhitzt. Unter Durchleitung von $CO_2$ wurde 9 Stunden nachgerührt und nach Filtrieren und Abkühlung 179 T (96,8 &) einer klaren gelben Flüssigkeit erhalten. Epoxidgehalt: 0,2 %, Siedepunkt: 136°C / 0,1 - 0,2 Torr.

24) 1420 T Glycidylmethacrylat, 13 T Triphenylphosphan und 5 T Natriumjodid wurden in einer Druckapparatur in Gegenwart von 3 T Hydrochinonmonomethylethylether bei 80°C 10 Stunden lang unter einem konstanten Kohlendioxiddruck von 1,5 bar gerührt. Nach Filtration wurden 1780 T (95,5 %) einer hellgelben viskosen Flüssigkeit erhalten. IR-Absorption: 1797, 1723 $cm^{-1}$, Epoxidgehalt: 0,3 %.

25) 260 T eines Glycidylesters der Versaticsäure (Epoxidgehalt: 6,2 %), 4,7 T Triphenylphosphan und 3,3 T Kaliumjodid wurden unter Durchleitung von Kohlendioxid 18 Stunden auf 120°C erhitzt. Ausbeute: 229 T (98,9 %) einer hellgelben viskosen Flüssigkeit. IR-Absorption: 1733, 1801 $cm^{-1}$, Epoxidgehalt: 0,25 %, Gehalt an gebundenem $CO_2$: 14,1 %.

26) 128 T Octen-1-oxid, 2,2 T Triphenylphosphan und 1,9 T Kaliumjodid wurden unter Durchleitung von Kohlendioxid auf 120°C erhitzt und bis zu einem Epoxidgehalt 0,2 % nachgerührt. Nach Filtration erhielt man 125 T (93,3 %) einer klaren gelben Flüssigkeit. IR-Absorption: 1800 $cm^{-1}$, Epoxidgehalt: 0,1 %, Gehalt an gebundenem $CO_2$: 22,9 %.

27) 330 T eines handelsüblichen glycidylethylethergruppenhaltigen Novolakes (DEN 731 der Fa. DOW Chemicals) (Epoxidgehalt: 9,1 %) wurden in 141 T Diglykoldimethylethers gelöst und unter gleichzeitigem Durchleiten von Kohlendioxid mit 1,2 T 4-Dimethylaminopyridin versetzt. Nach Erwärmen auf 100°C wurde unter fortlaufender Kohlendioxid-Einleitung bis zu einem Epoxidgehalt 0,1 % nachgerührt (Dauer ca. 16 Stunden). Nach Abdestillieren des Lösungsmittels unter vermindertem Druck wurden 380 T (92,4 %) eines klaren, farblösen, carbonatgruppenhaltigen Novolakes erhalten. Epoxidgehalt: < 0,1 %, Gehalt an gebundenem $CO_2$: 19,0 %.

28) 200 T eines handelsüblichen Glycidylethers ("®Beckopox EP 080 der Fa. Hoechst AG) (Epoxidgehalt: 8 %) wurden bei 25°C mit 0,5 T 2-Ethyl-4-methyl-imidazol versetzt und unter Durchleiten von Kohlendioxid auf 110°C erhitzt. Anschließend wurde bis zu einem Epoxidgehalt von < 0,3 % nachgerührt. Ausbeute: 218 (90,1 %) einer hellgelben viskosen Flüssigkeit. Epoxidgehalt: 0,3 %. Gehalt an geb. $CO_2$: 17,1 %.

29) 260 T eines handelsüblichen Triglycidylisocyanurats ("®Araldit PT 810" der Fa. Ciba-Geigy) wurden mit 260 T Diglykoldimethylether und 1 T 2-Ethyl-4-methylimidazol versetzt und unter Einhalten von Kohlendioxid auf 120°C erhitzt. Unter weiterem Einhalten von Kohlendioxid wurde bis zu einem Epoxidgehalt < 0,5 % nachgerührt, wobei ein weißes, kristallines Produkt ausfiel. Nach beendeter Reaktion wurde der Niederschlag abgesaugt und bei 60°C getrocknet. Ausbeute: 318 T (86,7 %) einer weißen, kristallinen Verbindung. Schmelzpunkt: 204 - 210°C (Zers.), Epoxidgehalt: 0,9 %, Gehalt an geb. $CO_2$: 27,4 %.

30) 300 T Polyethylenadipat (hergestellt aus 10 Mol Ethylenglykol und 11 Mol Adipinsäure durch azeotrope Veresterung, OH-Zahl: 56) wurden in 90 T Diethylenglykoldimethylether gelöst und auf 70°C erhitzt. Nach Zugabe von 0,1 T 4-Dimethylaminopyridin wurden portionsweise 44 T Phthalsäureanhydrid zugegeben. Nach Erreichen einer Säurezahl von 40 wurde der Ansatz mit 118 T eines technischen Diglycidylethers vom Bisphenol A-Typ (Beckopox EP 140, Epoxidgehalt 8,6 %) und mit 0,3 T Chrom-III-oktoats versetzt und auf 110°C erhitzt. Nach Erreichen einer Säurezahl 1 wurde nach Zugabe von 0,5 T 4-Dimethylaminopyridin und unter Einleiten von Kohlendioxid auf 120°C erhitzt und bis zu einem Epoxidgehalt < 0,05 % nachgerührt. Ausbeute: 550 T einer klaren, viskosen Harzlösung, Epoxidgehalt: 0,04 %, Gehalt an geb. $CO_2$: 2,0 %.

31) Die Reaktionsprodukte der Beispiele 15 und 21 (Vergleich 1) wurden gelpermeationschromatographisch auf ihre Zusammensetzung untersucht. Das Trägermaterial der Säule war ein Polystyrol der Fa. Waters Millipore unter der Bezeichnung Ultrastyragel (1000 - 100 Å, 1000 psi).

| Beispiel | %-Gehalt an Biscarbonat | Monocarbonat | Bisepoxid | Polymeranteil |
|---|---|---|---|---|
| 15 | 84,9 | 1,7 | - | 13,4 |
| 21 (v1) | 27,9 | 5,9 | - | 66,2 |

Die Untersuchung zeigt deutlich die Selektivität der erfindungsgemäßen Umsetzung Epoxidverbindungen mit $CO_2$. während hierbei der Polymeranteil gegenüber dem des eingesetzten technischen Epoxids praktisch unverändert bleibt, stieg der Polymerengehalt bei Verwendung von Triethylamin als Katalysator auf 66,2 %, bezogen auf die Gesamtmasse.

Wird der Triethylaminzusatz erhöht, z. B. auf 3 % wie in Beispiel 22 (Vergleich 2), geliert der Ansatz nach einer Stunde Reaktionsdauer.

## Patentansprüche

1. Verfahren zur Herstellung von 2-Oxo-1,3-dioxolanen durch Umsetzung von Epoxiden mit Kohlendioxid in Gegenwart eines Katalysators und gegebenenfalls eines inerten Lösungsmittels, dadurch gekennzeichnet, daß die Umsetzung bei Temperaturen von 40 bis 180°C und bei Normaldruck oder geringfügig erhöhtem Druck in Gegenwart mindestens eines Katalysators aus der Gruppe Triphenylphosphan, Tritolylphosphan, Diazabicyclo[2.2.2]octan, 4-Dimethyaminopyridin, 4-(1-Pyrrolidinyl)pyridin, Amidine, Imidazol und dessen Alkyl-, Aryl- oder Aralkyl-Substitutionsprodukten erfolgt.

2. Verfahren nach Anspruch 1, dadurch gekennzeichnet, daß bei einem Druck von 1 bis 5 bar gearbeitet wird.

3. Verfahren nach Anspruch 1 und/oder 2, dadurch gekennzeichnet, daß der Katalysator in Mengen von 0,02 bis 10 Gew.-% bezogen auf die Epoxidkomponente, eingesetzt wird.

4. Verfahren nach einem oder mehreren der Ansprüche 1 bis 3, dadurch gekennzeichnet, daß die Umsetzung der Epoxidverbindung mit Kohlendioxid nur teilweise vorgenommen wird.

5. Verfahren nach einem oder mehreren der Ansprüche 1 bis 4, dadurch gekennzeichnet, daß als Imidazole 1-Methyl-, 2-Methyl- und/oder 2-Ethyl-4-methyl-imidazol eingesetzt werden.

6. Verfahren nach einem oder mehreren Ansprüche 1 bis 5, dadurch gekennzeichnet, daß zusätzlich Co-Katalysatoren in Menge von 0,1 bis 10 Gew.-%, bezogen auf die Epoxidkomponente, eingesetzt werden.

7. Verfahren nach einem oder mehreren der Ansprüche 1 bis 6, dadurch gekennzeichnet, daß als Co-Katalysatoren Natrium- und/oder Kaliumjodid eingesetzt werden.

8. Verfahren nach einem oder mehreren der Ansprüche 1 bis 7, dadurch gekennzeichnet, daß die Epoxidverbindungen mindestend eine endständige 1,2-Epoxygruppe aufweisen.

9. Verfahren nach Anspruch 8, dadurch gekennzeichnet, daß als Epoxidverbindungen aliphatische Epoxide mit mindestens 6 C-Atomen, Glycidol, Epihalogenhydrine der Formel (2)

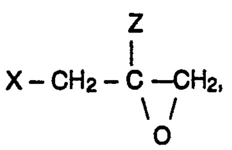

in der Z ein Wasserstoffatom, eine Methyl oder Ethylgruppe und X ein Halogenatom oder eine OH-Gruppe ist, eingesetzt werden

10. Verfahren nach Anspruch 8, dadurch gekennzeichnet, daß als Epoxidverbindungen solche eingesetzt werden, die im Durchschnitt mindestens eine substituierte oder nicht substituierte Glycidylethergruppe der Formel (3)

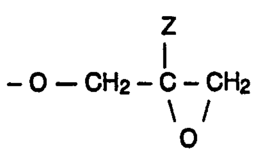

oder eine substituierte oder nicht substituierte Glycidylestergruppe der Formel (4)

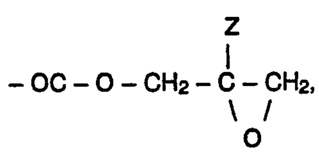

wobei in beiden Formeln Z die schon genannte Bedeutung hat, erhalten, ferner epoxydierte, mehrfach ungesättige Verbindungen und amid- oder urethangruppenhaltige Epoxide.

11. Verfahren nach einem der Ansprüche 8 bis 10, dadurch gekennzeichnet, daß als Epoxidverbindungen Polyglycidylether, plastifizierte Epoxidharze mit endständigen Epoxidgruppen oder Glycidylester von gesättigten oder ethylenisch ungesättigten (Poly) Carbonsäuren eingesetzt werden.

## Claims

1. A process for the preparation of 2-oxo-1,3-dioxolanes by reaction of epoxides with carbon dioxide in the presence of a catalyst and, if necessary, an inert solvent, wherein the reaction is effected at temperatures from 40 to 180°C and at normal pressure or slightly increased pressure in the presence of at least one catalyst from the group comprising triphenylphosphane, tritolylphosphane, diazabicyclo[2.2.2]octane, 4-dimethylaminopyridine, 4-(1-pyrrolidinyl)pyridine, amidines, imidazole and the alkyl, aryl, or aralkyl substitution products thereof.

2. The process as claimed in claim 1, wherein a pressure of 1 to 5 bar is employed.

3. The process as claimed in claim 1 and/or 2, wherein the catalyst is used in quantities of 0.02 to 10 % by weight, referred to the epoxy component.

4. The process as claimed in one or more of claims 1 to 3, wherein the reaction of the epoxy compound with carbon dioxide is only partially carried out.

5. The process as claimed in one or more of claims 1 to 4, wherein 1-methylimidazole, 2-methylimidazole and/or 2-ethyl-4-methylimidazole are used as imidazoles.

6. The process as claimed in one or more of claims 1 to 5, wherein cocatalysts are additionally used in quantities of 0.1 to 10 % by weight, referred to the epoxy component.

7. The process as claimed in one or more of claims 1 to 6, wherein sodium and/or potassium iodide are used as cocatalysts.

8. The process as claimed in one or more of claims 1 to 7, wherein the epoxy compounds have at least one terminal 1, 2-epoxy group.

9. The process as claimed in claim 8, wherein aliphatic epoxides with at least 6 carbon atoms, glycidol, or epihalohydrines of the formula (2)

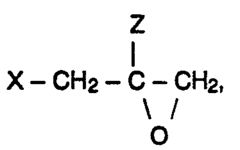

in which Z is a hydrogen atom, a methyl or ethyl group and X is a halogen atom or an OH group, are used as the epoxy compounds.

10. The process as claimed in claim 8, wherein those compounds which contain on average at least one substituted or unsubstituted glycidyl ether group of the formula (3)

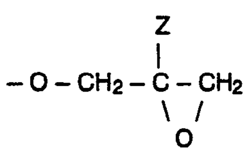

or a substituted or unsubstituted glycidyl ester group of the formula (4)

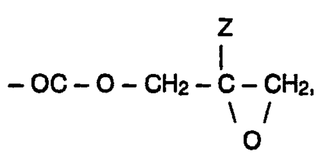

Z having the meaning already mentioned in both formulae, and furthermore epoxidized, polyunsaturated compounds and epoxides containing amide or urethane groups are used as epoxy compounds.

11. The process as claimed in one of claims 8 to 10, wherein polyglycidyl ether, plasticized epoxy resins with terminal epoxy groups or glycidyl esters of saturated or olefinically unsaturated (poly)carboxylic acids are used as epoxy compounds.

## Revendications

1. Procédé pour préparer des oxo-2 dioxolannes-1,3 par réaction d'époxydes avec le dioxyde de carbone, en présence d'un catalyseur et, éventuellement, d'un solvant inerte, procédé caractérisé en ce qu'on effectue la réaction à des températures de 40 à 180°C, sous la pression normale ou sous une pression légèrement élevée, en présence d'au moins un catalyseur pris dans l'ensemble constitué par le triphénylphosphane, le tritolylphosphane, le diazabicyclo[2.2.2]octane, la diméthylamino-4 pyridine, la (pyrrolidinyl-1)-4 pyridine, des amidines, l'imidazole et des imidazoles porteurs d'un radical alkyle, aryle ou aralkyle.

2. Procédé selon la revendication 1, caractérisé en ce qu'on opère sous une pression de 1 à 5 bar.

3. Procédé selon l'une des revendications 1 et 2, caractérisé en ce que le catalyseur est mis en jeu en des quantités de 0,02 à 10 % en poids par rapport à la composante époxydique.

4. Procédé selon l'une quelconque des revendications 1 à 3, caractérisé en ce que la réaction du composé époxydique avec le dioxyde de carbone n'est effectuée que partiellement.

5. Procédé selon l'une quelconque des revendications 1 à 4, caractérisé en ce qu'on utilise, comme imidazoles, les méthyl-1, méthyl-2 et/ou éthyl-2 méthyl-4 imidazoles.

6. Procédé selon l'une quelconque des revendications 1 à 5, caractérisé en ce qu'on utilise en outre des co-catalyseurs en des quantités de 0,1 à 10 % en poids par rapport à la composante époxydique.

7. Procédé selon l'une quelconque des revendications 1 à 6, caractérisé en ce qu'on utilise, comme co-catalyseur, l'iodure de sodium et/ou l'iodure de potassium.

8. Procédé selon l'une quelconque des revendications 1 à 7, caractérisé en ce que les composés époxydiques contiennent au moins un radical époxy-1,2 terminal.

9. Procédé selon la revendication 8, caractérisé en ce qu'on utilise, comme composés époxydiques, des époxydes aliphatiques à au moins 6 atomes de carbone, le glycidol ou des épihalogénhydrines répondant à la formule (2):

```
             Z
             |
X – CH2 – C – CH2,
             \ /
              O
```

dans laquelle Z représente un atome d'hydrogène ou un radical méthyle ou éthyle et X représente un atome d'halogène ou un radical OH.

10. Procédé selon la revendication 8, caractérisé en ce qu'on utilise, comme composés époxydiques, des composés de ce genre qui contiennent en moyenne au moins un radical d'éther glycidylique, substitué ou non, qui répond à la formule (3):

```
              Z
              |
– O – CH2 – C – CH2
              \ /
               O
```

ou un radical d'ester glycidylique, substitué ou non, qui répond à la formule (4):

```
                     Z
                     |
– OC – O – CH2 – C – CH2
                     \ /
                      O
```

formules dans lesquelles Z a la signification qui lui a déjà été donnée, des composés multi-insaturés époxydés, ou des époxydes contenant des radicaux d'amides ou d'uréthannes.

11. Procédé selon l'une quelconque des revendications 8 à 10, caractérisé en ce qu'on utilise, comme composés époxydiques, des éthers polyglycidyliques, des résines époxydiques plastifiées contenant des radicaux époxy terminaux, ou des esters glycidyliques d'acides (poly)carboxyliques saturés ou éthyléniques.